# EUROPEAN PATENT SPECIFICATION

(11) **EP 1 344 809 B1**
(45) Date of publication and mention of the grant of the patent: **17.11.2010**
(21) Application number: 03251068.7
(22) Date of filing: 21.02.2003
(51) Int. Cl.: C10L 1/183, C10L 1/188, C10L 1/238, C10L 1/24, C10L 1/30

(54) **Use of Dîesel Fuel Additive Compositions for Improvement of Particulate Traps**
Verwendung von Additivzusatzzusammensetzungen in Dieselkraftstoffen zur Verbesserung von Partikelfiltern
Utilisation de compositions d'addtifs pour des combustribles diesel afin d'améliorer les pièges à particules

(30) Priority: 13.03.2002 EP 02251757; 09.04.2002 EP 02252521
(43) Date of publication of application: 17.09.2003
(73) Proprietor: Infineum International Limited, Abingdon, Oxfordshire OX13 6BB (GB)
(72) Inventor: Collier, Philip Edward, Oxford OX24 2JR (GB); Caprotti, Rinaldo, Oxford OX2 9HD (GB)
(74) Representative: Lewis, Pauline Therese

(56) References cited:
- EP-A- 0 476 196
- WO-A-00/58422
- WO-A-02/10317
- WO-A-88/03551
- WO-A-97/44414
- US-A- 4 505 718
- US-A- 5 449 387

## Description

This invention relates to the use of a metal salt-ashless detergent additive composition which has been found highly effective in improving the quality of emissions from the combustion of diesel fuels. This additives composition is effective in improving the performance of particulate traps which are used in the exhaust systems of diesel engines, amongst other uses.

Diesel engines equipped with particulate traps, mounted in the exhaust stream, to "trap" or collect particulates in the exhaust to prevent their emission to the atmosphere are expected to be in greater use in the next few years.

Diesel engines running without particulate traps emit unburned hydrocarbons (HC), carbon monoxide (CO), nitrogen oxides (NOₓ), and particulates, all of which are subject to current or proposed regulation. The problems of controlling these pollutants are compounded because there is a trade-off between particulates and nitrogen oxides: when the combustion conditions are modified to favor low nitrogen oxides emissions, particulates are increased. Particulate traps are employed to reduce the severity of the particulate emissions.

It now appears that a combination of techniques, including diesel traps and systems that use nitrogen oxides, will be required to meet realistic clean air goals. This manner of reducing particulates will be necessary because the techniques available for NOₓ reduction, such as timing changes and exhaust gas recirculation, require a trade-off with particulates. The achievement of lower emissions of NOₓ, unburned hydrocarbons, and carbon monoxide, while controlling particulates over reasonable periods of time, continues to present a technical challenge.

Diesel particulates, their effect and control, are at the center of much concern and controversy. Their chemistry and environmental impact present complex issues. Generally, the diesel particulate matter is principally solid particles of carbon and metal compounds with adsorbed hydrocarbons, sulfates and aqueous species. Among the adsorbed species are aldehydes and polycyclic aromatic hydrocarbons. Some of these organics have been reported to be potential carcinogens or mutagens. Unburned hydrocarbons are related to the characteristic diesel odor and include aldehydes such as formaldehyde and acrolein. The need to control nano-particles is likely to lead to mandates requiring traps.

Unfortunately, increasing the recovery of particulates simply by modifying trap design or size would increase the rate of back pressure buildup within the trap, which causes increased fuel consumption and poor driveability. Moreover, control of the various pollutants seems to be interrelated, with reduction of one sometimes increasing levels of another. By modifying combustion to achieve more complete oxidation, decreases can be achieved for pollutants resulting from incomplete combustion, but NOₓ is typically increased under these conditions.

It is clear that diesel traps (either catalyzed or uncatalyzed) will be required in order to control particulates, especially where efforts are made to control NOₓ.

The use of diesel traps and the need to improve them has resulted in a great deal of research and a great number of patents and technical publications. The traps are typically constructed of metal or ceramic and are capable of collecting the particulates from the exhaust and withstanding the heat produced by oxidation of carbonaceous deposits which must be burned off at regular intervals.

This burning off, or regeneration, could occur by itself if the operating temperature of the trap were sufficiently high. However, in the typical situation, the exhaust temperature is not constantly high enough, and secondary measures such as electrically heating to raise the trap temperature or using a catalyst on the washcoat to reduce the combustion temperature of particulates, have not been fully successful.

The use of organometallic salts and complexes to improve the operation of diesel engine particulate traps is disclosed, for example, in U.S. Patent No. 5,344,467 issued September 6, 1994, which teaches the use of a combination of an organometallic complex and an antioxidant. The organometallic complex is soluble or dispersible in the diesel fuel and is derived from an organic compound containing at least two functional groups attached to a hydrocarbon linkage.

WO99/36488 published July 22, 1999 discloses fuel additive compositions which contain at least one iron-containing fuel-soluble or fuel-dispersible species in synergistic combination with at least one alkaline earth group metal-containing fuel-soluble or fuel-dispersible species. This combination of metallic additives is said to improve the operation of the diesel particulate filter traps.

Also pertinent to the subject matter of this invention is U.S. Patent No. 4,946,609 issued August 7, 1990, which teaches the use of iron compounds such as ferrocene, ferrocene derivatives and iron salts of organic acids as additives for lubricating oils used for diesel engines. It is taught that the presence of the iron compounds in the lubricating oil facilitates the regeneration of the diesel particle filters.

WO94/11467 published May 26, 1994 teaches a method to improve the operation of diesel traps through the use of a fuel additive comprising fuel-soluble compositions of a platinum group metal in effective amounts to lower the emissions of unburned hydrocarbons and carbon monoxide from the trap. The platinum group metals comprise platinum, palladium, rhodium or iridium.

US 4,505,718 describes combinations of transition metal salts of organic acids together with ashless hydrocarbon-soluble dispersants. These are used to treat lubricants and fuels.

EP 0 476 196 describes fuel additive composition which contain, as essential constituents, at least one fuel-soluble manganese carbonyl compound, at least one fuel-soluble alkali or alkaline earth metal-containing salt and at least one fuel-soluble ashless dispersant. The compositions may also contain other optional fuel additives. Improvements in fuel consumption are described.

WO 97/44414 relates to a process for reducing liner lacquering in marine diesel engines. The process comprises adding to the fuel at least one diesel detergent and at least one combustion improver. The detergent may be a succinimide species and the combustion improver is preferably a cerium oxidic compound.

WO 00/58422 relates to fuel oils which contain, as essential constituents, (a) at least one fuel-soluble or dispersible neutral alkaline earth metal compound and/or at least one fuel-soluble or dispersible neutral alkali metal compound, and (b) at least one fuel-soluble or dispersible transition metal compound. The fuel oils may also contain co-additives such as detergents and dispersants, e.g. polyisobutenyl succinimides. The specification describes that the fuel oils may improve the regenerative ability of particulate traps.

In accordance with the present invention there is provided the use of an additive composition as set out in claim 1.

Preferably, there is about 2-10 ppm of metal in the fuel, such as about 5-10 ppm metal, and 10-250 ppm of the ashless detergent additive such as about 10-25, 10-50, 50-200 ppm or 10-200 ppm of the ashless detergent (active ingredient basis).

Stable solutions or dispersions of the additive compositions of this invention in a suitable solvent comprise a further embodiment of this invention. Such additive concentrates will contain 20 to 80% of active material, which is the combination of the metal salt and ashless detergent. The active materials are present in the solvent in such amounts so as to provide in the fuel 1 to 25 ppm of metal and 10 to 500 ppm of ashless detergent. Such solutions or dispersions remain stable over a broad temperature range, especially solutions or dispersions containing iron or calcium.

The solvent used to prepare the stable additive solutions or dispersions may generally be characterized as a normally liquid petroleum or synthetic hydrocarbon or oxygenated hydrocarbon or alcohol solvents, such as hexanol, 2-ethylhexanol or isodecyl alcohol solvent. Typical examples include kerosene, hydrotreated kerosene, isoparaffinic and paraffinic solvents and naphthenic aliphatic hydrocarbon solvents, aromatic solvents, dimers and higher oligomers or propylene, butene and similar olefins and mixtures thereof. Commercial products such as "Solvesso", "Varsol", "Norpar" and "Isopar" are suitable. Such solvents may also contain functional groups other than carbon and hydrogen provided such groups do not adversely affect the performance of the additive composition. Preferred are isoparaffinic and paraffinic hydrocarbon solvents. Preferably, the solvent has a flash point greater than 20°C, more preferably greater than 40°C, most preferably greater than 55°C.

A wide variety of metals are suitable for forming the metal salt useful as additives in the present invention. The metal may be an alkali metal, preferably Na, an alkaline earth metal, such as Ca, Mg or Sr, a Group IVB metal, especially Ti or Zr, a Group VIIB metal, such as Mn, a Group VIII metal, particularly Fe, a Group IB metal, especially Cu, a Group IIB metal, such as Zn or any of the rare earth (lanthanide series of metals) metals having atomic numbers 57-71, especially cerium, or mixtures of any of the foregoing metals. The most preferred metal is iron.

It has been found that the additive composition greatly improves the operation of diesel exhaust particulate traps by lowering the ignition temperature of the particulates which build up in the trap, and this improvement is observed by noting the reduction in back pressure buildup when fuels containing the metal salt dispersant combination are used in the diesel engine. The improvements observed in accordance with the present invention are particularly surprising since it has been found that diesel fuels containing only the dispersant and not the metal salt will not improve the operation of the particulate traps and will in fact cause a greater back pressure buildup than what is observed with the same fuel containing no additive. Similarly, diesel fuels containing only the metal salt additive and not the ashless detergent do not provide as great an improvement in the performance of the diesel particulate traps.

In one embodiment of the invention the additive composition is admixed with the diesel fuel by direct addition, and the diesel fuel is used to operate a diesel engine equipped with an exhaust system particulate trap. The diesel fuel containing the composition is contained in a fuel tank, transmitted to the diesel engine where it is burned, and the composition reduces the ignition temperature of exhaust particles collected in the exhaust system particulate trap. In another embodiment, the foregoing operational procedure is used except that the additive composition is maintained on board the apparatus being powered by the diesel engine (e.g., automobile, bus, truck, etc.) in a separate fuel additive dispenser apart from the diesel fuel. The composition is combined or blended with the diesel fuel during re-filling of the diesel fuel tank. Typically, the additive is dispensed in the form of a solution in a hydrocarbon solvent. In this latter embodiment, the additive is maintained in the fuel additive dispenser and can form a part of a fuel additive concentrate of the concentrate being combined with the diesel. Other techniques comprise adding the additive composition into the intake manifold or adding the additive composition to the fuel at fuel depots prior to filling the tank of the diesel powered vehicle. Also, the metal salt may be added to a fuel which already has the ashless detergent present. However, the fuels compositions embodiments of the present invention do not extend to fuels compositions containing a metal salt where the metal salt is a mixture composed of one or more cerium fatty acid carboxylates, such as cerium octadecanoate or other cerium C8-C22 fatty acid carboxylates, and 50% or more cerium oxide, unless such mixture has been added to the fuel as part of an additive combination with ashless detergent.

A category of metal salts useful in this invention are those which comprise a polar head with long hydrophobic tail, with the polar head comprising a metal salt of an acid organic compound. The salts may contain a substantially stoichiometric amount of the metal in which they are usually described as normal or neutral salts, and would typically have a total base number (TBN), as may be measured by ASTM D-2896 of from 0 to 80. It is possible to include large amounts of a metal base by reacting an excess of a metal compound such as an oxide or hydroxide with an acid gas such as carbon dioxide. The resulting overbased salt comprises neutralized salt as the outer layer of a metal base (e.g., carbonate) micelle. Such overbased salts may have a TBN of 150 or greater, and typically from 250 to 600, such as in the range of from 350 to 450 TBN. Overbasing for all metals useful in this invention may be from the use of metal oxide.

The salt may be neutral in that it contains a stoichiometric ratio of metal cations to carboxylate anions. It may also be acidic or overbased. Acidic salts contain an excess of carboxylic acid/carboxylate over that which would be considered stoichiometric and overbased salts contains an excess of metal species over the stoichiometric ratio. This excess metal may exist in one or a combination of forms including oxide, hydroxides or mixed oxidic salts. Lattice-like polynuclear-metal complexes may also be present.

For overbased salts, the excess metal may be introduced, either intentionally or unintentionally, during the main reaction process or alternatively may be introduced subsequent to this via post treatment. The elemental metal, oxides and hydroxides are common feedstocks for the overbasing process.

Metal salts that may be used include oil soluble or dispersible neutral and overbased sulfonates, phenates, sulfurized phenates, thiophosphonates, salicylates, and naphthenates and other oil-soluble carboxylates of a metal, particularly iron, cerium and the alkali or alkaline earth metals, e.g., sodium, potassium, lithium, calcium, and magnesium. The most commonly used metals are iron, cerium, calcium and magnesium, and mixtures of calcium and/or magnesium with sodium. Particularly convenient metal salts are neutral and overbased calcium sulfonates having TBN of from 20 to 600 TBN, and neutral and overbased calcium phenates and sulfurized phenates having TBN of from 50 to 600 as well as iron neodecanoates and naphthenates. Sulfonates, salicylates and naphthenates are preferred. The metal salts may also be acidic, i.e., it may contain up to 20% of unreacted free acid such as 1-20% by weight free acid.

The organic moiety of the metal salt compounds preferably contain at least one hydrocarbyl group, for example, as a substituent on an aromatic ring. The term "hydrocarbyl" as used herein means that the group concerned is primarily composed of hydrogen and carbon atoms and is bonded to the remainder of the molecule via a carbon atom but does not exclude the presence of other atoms or groups in a proportion insufficient to detract from the substantially hydrocarbon characteristics of the group. Advantageously, hydrocarbyl groups for use in accordance with the invention are aliphatic groups, preferably alkyl or alkylene groups, especially alkyl groups, which may be linear or branched. The total number of carbon atoms in the detergents should be at least sufficient to impart the desired oil-solubility.

Phenols, for use in the metal salts of this invention, may be non-sulfurized or, preferably, sulfurized. Further, the term "phenol" as used herein includes phenols containing more than one hydroxyl group (for example, alkyl catechols) or fused aromatic rings (for example, alkyl naphthols) and phenols which have been modified by chemical reaction, for example, alkylene-bridged phenols and Mannich base-condensed phenols; and saligenin-type phenols (produced by the reaction of a phenol and an aldehyde under basic conditions).

Preferred phenols may be derived from the formula 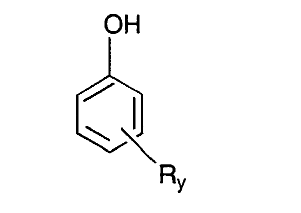 where R represents a hydrocarbyl group and y represents 1 to 4. Where y is greater than 1, the hydrocarbyl groups may be the same or different.

The phenols are frequently used in sulfurized form. Sulfurized hydrocarbyl phenols may typically be represented by the formula: 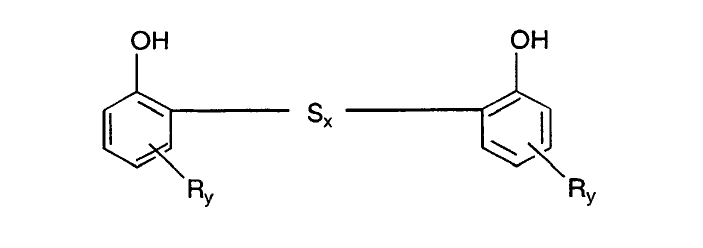 where x is generally from 1 to 4. In some cases, more than two phenol molecules may be linked by Sₓ bridges.

In the above formulae, hydrocarbyl groups represented by R are advantageously alkyl groups, which advantageously contain 5 to 100, preferably 5 to 40, especially 9 to 12, carbon atoms, the average number of carbon atoms in all of the R groups being at least 9 in order to ensure adequate solubility in oil. Preferred alkyl groups are nonyl (tripropylene) groups.

In the following discussion, hydrocarbyl-substituted phenols will for convenience be referred to as alkyl phenols.

A sulfurizing agent for use in preparing a sulfurized phenol or phenate may be any compound or element which introduces -(S)ₓ- bridging groups between the alkyl phenol monomer groups, wherein x is generally from 1 to about 4. Thus, the reaction may be conducted with elemental sulfur or a halide thereof, for example, sulfur dichloride or, more preferably, sulfur monochloride. If elemental sulfur is used, the sulfurization reaction may be effected by heating the alkyl phenol compound at from 50 to 250, preferably at least 100, °C. The use of elemental sulfur will typically yield a mixture of bridging groups -(S)x- as described above. If a sulfur halide is used, the sulfurization reaction may be effected by treating the alkyl phenol at from -10 to 120, preferably at least 60, °C. The reaction may be conducted in the presence of a suitable diluent. The diluent advantageously comprises a substantially inert organic diluent, for example mineral oil or an alkane. In any event, the reaction is conducted for a period of time sufficient to effect substantial reaction. It is generally preferred to employ from 0.1 to 5 moles of the alkyl phenol material per equivalent of sulphurizing agent.

Where elemental sulfur is used as the sulfurizing agent, it may be desirable to use a basic catalyst, for example, sodium hydroxide or an organic amine, preferably a heterocyclic amine (e.g., morpholine).

Details of sulfurization processes are well known to those skilled in the art.

Regardless of the manner in which they are prepared, sulfurized alkyl phenols useful in preparing overbased metal compounds generally comprise diluent and unreacted alkyl phenols and generally contain from 2 to 20, preferably 4 to 14, and most preferably 6 to 12, mass % sulfur based on the mass of the sulfurized alkyl phenol.

As indicated above, the term "phenol" as used herein includes phenols that have been modified by chemical reaction with, for example, an aldehyde, and Mannich base-condensed phenols.

Aldehydes with which phenols may be modified include, for example, formaldehyde, propionaldehyde and butyraldehyde. The preferred aldehyde is formaldehyde. Aldehyde-modified phenols suitable for use are described in, for example, US-A-5 259 967.

Mannich base-condensed phenols are prepared by the reaction of a phenol, an aldehyde and an amine. Examples of suitable Mannich base-condensed phenols are described in GB-A-2 121 432.

In general, the phenols may include substituents other than those mentioned above provided that such substituents do not detract significantly from the surfactant properties of the phenols. Examples of such substituents are methoxy groups and halogen atoms.

Salicylic acids used for salicylate salts of the invention may be non-sulfurized or sulfurized, and may be chemically modified and/or contain additional substituents, for example, as discussed above for phenols. Processes similar to those described above may also be used for sulfurizing a hydrocarbyl-substituted salicylic acid, and are well known to those skilled in the art. Salicylic acids are typically prepared by the carboxylation, by the Kolbe-Schmitt process, of phenoxides, and in that case, will generally be obtained (normally in a diluent) in admixture with uncarboxylated phenol.

Preferred substituents in oil-soluble salicylic acids from which overbased detergents in accordance with the invention may be derived are the substituents represented by R in the above discussion of phenols. In alkyl-substituted salicylic acids, the alkyl groups advantageously contain 5 to 100, preferably 9 to 30, especially 14 to 20, carbon atoms.

Sulfonic acids used for metal sulfonate salts of this invention are typically obtained by sulfonation of hydrocarbyl-substituted, especially alkyl-substituted, aromatic hydrocarbons, for example, those obtained from the fractionation of petroleum by distillation and/or extraction, or by the alkylation of aromatic hydrocarbons. Examples include those obtained by alkylating benzene, toluene, xylene, naphthalene, biphenyl or their halogen derivatives, for example, chlorobenzene, chlorotoluene or chloronaphthalene. Alkylation of aromatic hydrocarbons may be carried out in the presence of a catalyst with alkylating agents having from 3 to more than 100 carbon atoms, such as, for example, haloparaffins, olefins that may be obtained by dehydrogenation of paraffins, and polyolefins, for example, polymers of ethylene, propylene, and/or butene. The alkylaryl sulphonic acids usually contain from 7 to 100 or more carbon atoms. They preferably contain from 16 to 80, or 12 to 40, carbon atoms per alkyl-substituted aromatic moiety, depending on the source from which they are obtained.

When neutralizing these alkylaryl sulfonic acids to provide sulfonates, hydrocarbon solvents and/or diluent oils may also be included in the reaction mixture, as well as promoters and viscosity control agents.

Another type of sulfonic acid that may be used comprises alkyl phenol sulfonic acids. Such sulfonic acids can be sulfurized. Whether sulfurized or non-sulfurized these sulfonic acids are believed to have surfactant properties comparable to those of sulfonic acids, rather than surfactant properties comparable to those of phenols.

Suitable sulfonic acids also include alkyl sulfonic acids, such as alkenyl sulfonic acids. In such compounds the alkyl group suitably contains 9 to 100, advantageously 12 to 80, especially 16 to 60, carbon atoms.

Carboxylic acids that may be used in accordance with the invention include mono- and dicarboxylic acids. Preferred monocarboxylic acids are those containing 1 to 30, especially 8 to 24, carbon atoms. (Where this specification indicates the number of carbon atoms in a carboxylic acid, the carbon atom(s) in the carboxylic group(s) is/are included in that number.) Examples of monocarboxylic acids are iso-octanoic acid, stearic acid, oleic acid, palmitic acid and behenic acid. Other examples are tall oil fatty acid, soy acid and acid derived from rapeseed oil. Iso-octanoic acid may, if desired, be used in the form of the mixture of C₈ acid isomers sold by ExxonMobil Chemical Co. under the trade name "Cekanoic". Other suitable acids are those with tertiary substitution at the α-carbon atom and dicarboxylic acids with more than 2 carbon atoms separating the carboxylic groups. Further, dicarboxylic acids with more than 35, for example, 36 to 100, carbon atoms are also suitable. Unsaturated carboxylic acids can be sulphurized. Although salicylic acids contain a carboxylic group, for the purposes of the present invention they are considered to be a separate group of surfactants, and are not considered to be carboxylic acid surfactants. (Nor, although they contain a hydroxyl group, are they considered to be phenol surfactants.)

Other acids are those formed by dimerizing fatty acids such as C₃₆ dimer acid and C₁₂-C₂₄ succinic anhydride hydrolysis products or acids derived from polyalkenylmaleic anhydride reaction products.

Examples of other compounds which may be used to provide metal salts for use in the invention include the following compounds, and derivatives thereof: naphthenic acids, especially naphthenic acids containing one or more alkyl groups, dialkylphosphonic acids, dialkylthiophosphonic acids, and dialkyldithiophosphoric acids, high molecular weight (preferably ethoxylated) alcohols, dithiocarbamic acids, thiophosphines, and dispersants. Surfactants of these types are well known to those skilled in the art. Surfactants of the hydrocarbyl-substituted carboxylalkylene-linked phenol type, or dihydrocarbyl esters of alkylene dicarboxylic acids, the alkylene group being substituted with a hydroxy group and an additional carboxylic acid group, or alkylene-linked polyaromatic molecules, the aromatic moieties whereof comprise at least one hydrocarbyl-substituted phenol and at least one carboxy phenol, may also be suitable for use in the present invention; such surfactants are described in EP-A-708 171.

As used in this specification the term "hydrocarbyl" refers to a group having a carbon atom directly attached to the rest of the molecule and having a hydrocarbon or predominantly hydrocarbon character. Examples include hydrocarbon groups, including aliphatic (e.g. alkyl or alkenyl), alicyclic (e.g. cycloalkyl or cycloalkenyl), aromatic, and alicyclic-substituted aromatic, and aromatic-substituted aliphatic and alicyclic groups. Aliphatic groups are advantageously saturated. These groups may contain non-hydrocarbon substituents provided their presence does not alter the predominantly hydrocarbon character of the group. Examples include keto, halo, hydroxy, nitro, cyano, alkoxy and acyl. If the hydrocarbyl group is substituted, a single (mono) substituent is preferred.

Preferred are metal salts, especially iron or cerium salts or complexes derived from an acid compound of the formula 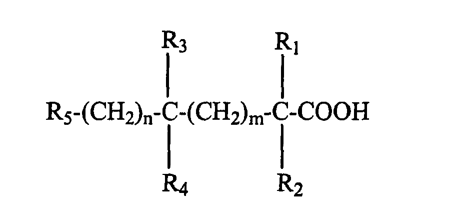 where R₁, R₂, R₃ and R₄ represent hydrogen or a hydrocarbyl having 1-30 carbon atoms (C₁-C₃₀), but at least two of R₁, R₂, R₃ or R₄ are C₁-C₃₀ hydrocarbyl; R₅ is a

hydrocarbyl having 1 to 120 carbon atoms and m and n may each be zero or an integer such that the total number of carbon atoms in the carboxylate is not more than 125. The formula above is intended to represent a carboxylic acid which has at least two side chains of at least 1 to 30 carbon atoms in length, and preferably both R₁ and R₂ are hydrocarbyl so that the carboxylate is a neocarboxylate, i.e., having the carbon atom which is alpha to the carbonyl carbon connected to four other carbon atoms. The term hydrocarbyl is intended to apply to aromatic or aliphatic radicals composed principally of carbon and hydrogen, optionally substituted with oxygen or nitrogen, preferably aliphatic and particularly straight or branched chain alkyl or substituted alkyl, the substituents being nitrogen or oxygen. Most preferably the carboxylate is a neodecanoate.

Suitable examples of R₅ moieties are hydrocarbyl groups are made from homo- or interpolymers (e.g. copolymers, terpolymers) of mono- and di-olefins having 2 to 10 carbon atoms, such as ethylene, propylene, 1-butene, isobutene, butadiene, isoprene, 1-hexene, 1-octene, etc. Typically, these olefins are 1-monoolefins. This hydrocarbyl can also be derived from the halogenated (e.g. chlorinated or brominated) analogs of such homo- or interpolymers or from polyethers.

The hydrocarbyl is predominantly saturated. The hydrocarbyl is predominantly aliphatic in nature, that is, containing no more than one non-aliphatic moiety (cycloalkyl, cycloalkenyl or aromatic) group of 6 or less carbon atoms for every 10 carbon atoms in the substituent. Usually, however, the hydrocarbyl contains no more than one such non-aliphatic group for every 50 carbon atoms, and in many cases, they contain no such non-aliphatic groups at all; that is, the typical substituents are purely aliphatic. Typically, these purely aliphatic hydrocarbyls are alkyl or alkenyl groups.

A preferred source of the R₅ moiety are poly(isobutene)s obtained by polymerization of a C₄ refinery stream having a butene content of 35 to 75 wt.% and isobutene content of 30 to 60 wt.% in the presence of a Lewis acid catalyst such as aluminum trichloride or boron trifluoride. These polybutenes predominantly contain monomer repeating units of the configuration -C(CH₃)₂CH₂-.

Iron neocarboxylate salts are preferred. The iron salt may be Fe+2 or Fe+3 salts or a mixture thereof. The iron salt may also contain ferrous or ferric oxide, which results from the process used to prepare the neocarboxylic acid iron salt, and when iron oxide is present the salt may be considered as overbased.

The metal salt or complex of the carboxylic acid may also be acidic, that is, the metal carboxylate or metal complex additive salt composition may contain up to about 20% of unreacted free acid such as 1-20% by weight free acid, more preferably 0-10%, most preferably 0-5% free acid.

The metal carboxylate or complex of the carboxylic acid may be overbased, as discussed earlier, or neutral but preferably is neutral.

The metal salt may be an oil soluble or dispersible ferrocene derivative, particularly a 2,2-bisferrocenylakane. Such materials are preferably alkane-bridged between the two ferrocenyl residues in which the alkane includes 1-8 carbon atoms and the four cyclopentadienyl rings carry independently of each other at least one alkyl group including 1-4 carbon atoms as substituents.

One class of nitrogen containing ashless detergents comprises an acylated nitrogen compound, preferably having a hydrocarbyl substituent of at least 10 aliphatic carbon atoms, made by reacting a carboxylic acid acylating agent with at least one amine compound containing at least one -NH-group, said acylating agent being linked to said amino compound through an imido, amido, amidine or acyloxy ammonium linkage, the ratio of hydrocarbyl units to amine units being 1:1 to 2.5:1, preferably 1.2:1 to 1.5:1.

Another class of nitrogen containing ashless detergents comprises the "polyalkylene amines". These are derived from polyalkylenes of greater than 250 mass units, which are themselves preferably derived from C₂-C₁₀ alkenes and more preferably from butene and/or isobutene. They are prepared by linking ammonia, amines, polyamines, alkylamines or alkanolamines to and/or between these polymers. A variety of methods can be used to achieve this, for example routes via chlorination, hydroformylation, epoxidation and ozonolysis are known in the art. Typical examples, which are also well known in the art, are polyisobutene monoamine ("PIBA") and polyisobutene-ethylenediamine ("PIB-EDA"). Further examples are described in EP 244616 and WO 98/28346. The ratio of hydrocarbyl units to amine units is 1:1 to 2.5:1, preferably 1.2:1 to 1.5:1. A number of acylated, nitrogen-containing compounds having a hydrocarbyl substituent of at least 10 carbon atoms and made by reacting a carboxylic acid acylating agent, for example an anhydride or ester, with an amino compound are known to those skilled in the art. In such compositions the acylating agent is linked to the amino compound through an imido, amido, amidine or acyloxy ammonium linkage. The hydrocarbyl substituent of 10 carbon atoms may be found either in the portion of the molecule derived from the carboxylic acid acylating agent, or in the portion derived from the amino compound, or in both. Preferably, however, it is found in the acylating agent portion. The acylating agent can vary from formic acid and its acylating derivatives to acylating agents having high molecular weight hydrocarbyl substituents of up to 50, 100 or 200 carbon atoms. The amino compounds can vary from ammonia itself to amines having hydrocarbyl substituents of up to about 30 carbon atoms.

A preferred class of acylated amino compounds are those made by reacting an acylating agent having a hydrocarbyl substituent of at least 10 carbon atoms and a nitrogen compound characterized by the presence of at least one -NH- group. Typically, the acylating agent will be a mono- or polycarboxylic acid (or reactive equivalent thereof) such as a substituted succinic or propionic acid and the amino compound will be a polyamine or mixture of polyamines, most typically, a mixture of ethylene polyamines. The amine also may be a hydroxyalkyl-substituted polyamine. The hydrocarbyl substituent in such acylating agents preferably averages at least about 30 or 50 and up to about 400 carbon atoms.

Illustrative of hydrocarbyl substituent groups containing at least 10 carbon atoms are n-decyl, n-dodecyl, tetrapropenyl, n-octadecyl, oleyl, chlorooctadecyl, triicontanyl, etc. Generally, the hydrocarbyl substituents are made from homo- or interpolymers (e.g. copolymers, terpolymers) of mono- and di-olefins having 2 to 10 carbon atoms, such as ethylene, propylene, 1-butene, isobutene, butadiene, isoprene, 1-hexene, 1-octene, etc. Typically, these olefins are 1-monoolefins. This substituent can also be derived from the halogenated (e.g. chlorinated or brominated) analogs of such homo-or interpolymers.

The hydrocarbyl substituents are predominantly saturated. The hydrocarbyl substituents are also predominantly aliphatic in nature, that is, they contain no more than one non-aliphatic moiety (cycloalkyl, cycloalkenyl or aromatic) group of 6 or less carbon atoms for every 10 carbon atoms in the substituent. Usually, however, the substituents contain no more than one such non-aliphatic group for every 50 carbon atoms, and in many cases, they contain no such non-aliphatic groups at all; that is, the typically substituents are purely aliphatic. Typically, these purely aliphatic substituents are alkyl or alkenyl groups.

A preferred source of the substituents are poly(isobutene)s obtained by polymerization of a C₄ refinery stream having a butene content of 35 to 75 weight per cent and isobutene content of 30 to 60 weight per cent in the presence of a Lewis acid catalyst such as aluminum trichloride or boron trifluoride. These polybutenes predominantly contain monomer repeating units of the configuration -C(CH₃)₂CH₂-.

The hydrocarbyl substituent is attached to the succinic acid moiety or derivative thereof via conventional means, for example the reaction between maleic anhydride and an unsaturated substituent precursor such as a polyalkene, as described for example in EP-B-0 451 380.

One procedure for preparing the substituted succinic acylating agents involves first chlorinating the polyalkene until there is an average of at least about one chloro group for each molecule of polyalkene. Chlorination involves merely contacting the polyalkene with chlorine gas until the desired amount of chlorine is incorporated into the chlorinated polyalkene. Chlorination is generally carried out at a temperature of about 75°C to about 125°C. If desired, a diluent can be used in the chlorination procedure. Suitable diluents for this purpose include poly- and perchlorinated and/or fluorinated alkanes and benzenes.

The second step in the procedure is to react the chlorinated polyalkene with the maleic reactant at a temperature usually within the range of about 100°C to about 200°C. The mole ratio of chlorinated polyalkene to maleic reactant is usually about 1:1. However, a stoichiometric excess of maleic reactant can be used, for example, a mole ratio of 1:2. If an average of more than about one chloro group per molecule of polyalkene is introduced during the chlorination step, then more than one mole of maleic reactant can react per molecule of chlorinated polyalkene. It is normally desirable to provide an excess of maleic reactant; for example, an excess of about 5% to about 50%, for example 25% by weight. Unreacted excess maleic reactant may be stripped from the reaction product, usually under vacuum.

Another procedure for preparing substituted succinic acid acylating agents utilizes a process described in U.S. Pat. No. 3,912,764 and U.K. Pat. No. 1,440,219. According to that process, the polyalkene and the maleic reactant are first reacted by heating them together in a direct alkylation procedure. When the direct alkylation step is completed, chlorine is introduced into the reaction mixture to promote reaction of the remaining unreacted maleic reactants. According to the patents, 0.3 to 2 or more moles of maleic anhydride are used in the reaction for each mole of polyalkene. The direct alkylation step is conducted at temperatures to 180°C to 250°C. During the chlorine-introducing stage, a temperature of 160°C to 225°C is employed.

The attachment of the hydrocarbyl substituent to the succinic moiety may alternatively be achieved via the thermally-driven 'ene' reaction, in the absence of chlorine. Use of such a material is the acylating agent (i) leads to products having particular advantages; for example, chlorine-free products having excellent detergency and lubricity properties. In such products, the reactant (i) is preferably formed from a polyalkene having at least 30% preferably 50% or more such as 75% of residual unsaturation in the form of terminal, e.g. vinylidene, double bonds.

The polyamines suitable in this invention are those comprising amino nitrogens linked by alkylene bridges, which amino nitrogens may be primary, secondary and/or tertiary in nature. The polyamines may be straight chain, wherein all the amino groups will be primary or secondary groups, or may contain cyclic or branched regions or both, in which case tertiary amino groups may also be present. The alkylene groups are preferably ethylene or propylene groups, with ethylene being preferred. Such materials may be prepared from the polymerization of lower alkylene diamines such as ethylene diamine, a mixture of polyamines being obtained, or via the reaction of dichloroethane and ammonia.

Specific examples of the polyalkylene polyamines (1) are ethylene diamine, tetra(ethylene)pentamine, tri-(trimethylene)tetramine, and 1,2-propylene diamine. Specific examples of hydroxyalkyl-substituted polyamines include N-(2-hydroxyethyl) ethylene diamine, N,N¹-bis-(2-hydroxyethyl) ethylene diamine, N-(3-hydroxybutyl) tetramethylene diamine, etc. Specific examples of the heterocyclic-substituted polyamines (2) are N-2-aminoethyl piperazine, N-2 and N-3 amino propyl morpholine, N-3-(dimethylamino) propyl piperazine, 2-heptyl-3-(2-aminopropyl) imidazoline, 1,4-bis (2-aminoethyl) piperazine, 1-(2-hydroxy ethyl) piperazine, and 2-heptadecyl-1-(2-hydroxyethyl)-imidazoline, etc. Specific examples of the aromatic polyamines (3) are the various isomeric phenylene diamines, the various isomeric naphthalene diamines, etc.

Many patents have described useful acylated nitrogen compounds including US Patents 3 172 892; 3 219 666; 3 272 746; 3 310 492; 3 341 542; 3 444 170; 3 455 831; 3 455 832; 3 576 743; 3 630 904; 3 632 511; 3 804 763 and 4 234 435, and including European patent applications EP 0 336 664 and EP 0 263 703. A typical and preferred compound of this class is that made by reacting a poly(isobutylene)-substituted succinic anhydride acylating agent (e.g. anhydride, acid, ester, etc.) wherein the poly(isobutene) substituent has between about 50 to about 400 carbon atoms with a mixture of ethylene polyamines having 3 to about 7 amino nitrogen atoms per ethylene polyamine and about 1 to about 6 ethylene groups. In view of the extensive disclosure of this type of acylated amino compound, further discussion of their nature and method of preparation is not needed here. The above-noted US patents are utilized for their disclosure of acylated amino compounds and their method of preparation.

Preferred materials also include those made from amine mixtures comprising polyamines having seven and eight, and optionally nine, nitrogen atoms per molecule (so-called 'heavy' polyamines).

More preferably, the polyamine mixture comprises at least 45% and preferably 50% by weight of polyamines having seven nitrogen atoms per molecule, based on the total weight of polyamines.

The polyamine component (ii) may be defined by the average number of nitrogen atoms per molecule of the component (ii), which may preferably be in the range of 4 to 8.5, more preferably 6.8 to 8, especially 6.8 to 7.5 nitrogens per molecule. The number of nitrogens appears to influence the ability of the product to provide deposit control.

Another type of acylated nitrogen compound belonging to this class is that made by reacting the afore-described alkylene amines with the afore-described substituted succinic acids or anhydrides and aliphatic mono-carboxylic acids having from 2 to about 22 carbon atoms. In these types of acylated nitrogen compounds, the mole ratio of succinic acid to mono-carboxylic acid ranges from about 1:0.1 to about 0.1:1, such as 1:1. Typical of the mono-carboxylic acid are formic acid, acetic acid, dodecanoic acid, butanoic acid, oleic acid, stearic acid, the commercial mixture of stearic acid isomers known as isosteric acid, tolyl acid, etc. Such materials are more fully described in US patents 3 216 936 and 3 250 715.

Still another type of acylated nitrogen compound is the product of the reaction of a fatty monocarboxylic acid of about 12-30 carbon atoms and the afore-described alkylene amines, typically, ethylene, propylene or trimethylene polyamines containing 2 to 8 amino groups and mixtures thereof. The fatty mono-carboxylic acids are generally mixtures of straight and branched chain fatty carboxylic acids containing 12-30 carbon atoms. A widely used type of acylating nitrogen compound is made by reacting the afore-described alkylene polyamines with a mixture of fatty acids having from 5 to about 30 mole per cent straight chain acid and about 70 to about 95 mole per cent branched chain fatty acids. Among the commercially available mixtures are those known widely in the trade as isostearic acid. These mixtures are produced as by-product from the dimerization of unsaturated fatty acids as described in US patents 2 812 342 and 3 260 671.

The preferred acylated nitrogen ashless detergent compounds are those made by reacting a poly (isobutene) substituted succinic anhydride acylating agent with mixtures of ethylene polyamines as hereinbefore described, wherein the polyisobutene has a Mn of about 400-2500, preferably 700-400, such as about 950.

Any fuel having a boiling range and viscosity suitable for use in a diesel-type compression ignition engine may be used in this invention.

Such fuel oils include "middle distillate" fuel oil which refers to petroleum-based fuel oils obtainable in refining crude oil as the fraction from the light, kerosene or jet fuel, fraction to the heavy fuel oil fraction. These fuel oils may also comprise atmospheric or vacuum distillate, cracked gas oil or a blend, in any proportions, of straight run and thermally and/or catalytically cracked distillate. Examples include kerosene, jet fuel, diesel fuel, heating oil, visbroken gas oil, light cycle oil and vacuum gas oil. Such middle distillate fuel oils usually boil over a temperature range, generally within the range of 100°C to 500°C, as measured according to ASTM D86, more especially between 150°C and 400°C. Preferably, the diesel fuel will have less than 0.1% by weight sulfur, more preferably less than 0.05% by weight sulfur as determined by ASTM D 2622-87.

Preferred vegetable-based fuel oils are triglycerides of monocarboxylic acids, for example, acids containing 10-25 carbon atoms, and typically have the general formula shown below 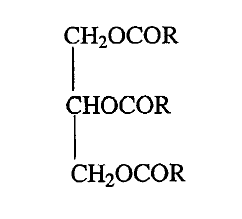 where R is an aliphatic radical of 10-25 carbon atoms which may be saturated or unsaturated.

Generally, such oils contain glycerides of a number of acids, the number and kind varying with the source vegetable of the oil.

Suitable fuel oils also include mixtures of 1-50% by weight of vegetable oils or methylesters of fatty acid, such as tall oil fatty acids with petroleum based diesel fuel oils. Also suitable are fuels emulsified with water and alcohols, which contain suitable surfactants, and residual fuel oil used in marine diesel engines.

Examples of oils are tall oil, rapeseed oil, coriander oil, soyabean oil, cottonseed oil, sunflower oil, castor oil, olive oil, peanut oil, maize oil, almond oil, palm kernel oil, coconut oil, mustard seed oil, beef tallow and fish oils. Rapeseed oil, which is a mixture of fatty acids partially esterified with glycerol, is preferred as it is available in large quantities and can be obtained in a simple way by pressing from rapeseed.

Further preferred examples of vegetable-based fuel oils are alkyl esters, such as methyl esters, of fatty acids of the vegetable or animal oils. Such esters can be made by transesterification.

As lower alkyl esters of fatty acids, consideration may be given to the following, for example as commercial mixtures: the ethyl, propyl, butyl and especially methyl esters of fatty acids with 12 to 22 carbon atoms, for example of lauric acid, myristic acid, palmitic acid, palmitoleic acid, stearic acid, oleic acid, elaidic acid, petroselic acid, ricinoleic acid, elaeostearic acid, linoleic acid, linolenic acid, eicosanoic acid, gadoleic acid, docosanoic acid or erucic acid, which have an iodine number from 50 to 150, especially 90 to 125. Mixtures with particularly advantageous properties are those which contain mainly, i.e. to at least 50 wt % methyl esters of fatty acids with 16 to 22 carbon atoms and 1, 2 or 3 double bonds. The preferred lower alkyl esters of fatty acids are the methyl esters of oleic acid, linoleic acid, linolenic acid and erucic acid.

Commercial mixtures of the stated kind are obtained for example by cleavage and esterification of natural fats and oils by their transesterification with lower aliphatic alcohols. For production of lower alkyl esters of fatty acids it is advantageous to start from fats and oils with high iodine number, such as, for example, sunflower oil, rapeseed oil, coriander oil, castor oil, soyabean oil, cottonseed oil, peanut oil or beef tallow. Lower alkyl esters of fatty acids based on a new variety of rapeseed oil, the fatty acid component of which is derived to more than 80 wt % from unsaturated fatty acids with 18 carbon atoms, are preferred.

Most preferred as a vegetable-based fuel oil is rapeseed methyl ester.

### Examples

A low sulfur diesel fuel containing less than 500 ppm sulfur was tested in a Ford 2.5 liter diesel engine equipped with a ceramic diesel particulate exhaust trap. The fuel was tested at 450°C (exhaust temperature prior to the trap) without additive (Base Fuel), with 5 ppm calcium from neutral calcium sulfonate (Fuel A), with 200 ppm Mn 950 polyisobutenyl succinimide (used as 55 wt.% solution of active ingredient) (Fuel B), with 1000 ppm of the same succinimide (Fuel C) and with a combination of 5 ppm calcium from the same sulfonate and 200 ppm of the same succinimide (Fuel D). The diesel exhaust back pressure was measured in mbar and is reported for each fuel over a period of several hours in the Table below. Fuel D, the fuel of this invention, clearly exhibits a significant improvement in back pressure reduction and particulate trap functioning. The control of back pressure increase has a positive effect on fuel consumption and this extends the life of the trap.

**Table - Back Pressure (mbar)**

| Time, Hours | Base Fuel | Fuel A | Fuel B | Fuel C | Fuel D |
|---|---|---|---|---|---|
| 0 | 80.4 | 140.0 | 80.4 | 79.9 | 80.6 |
| 0.5 | 111.4 | 152.0 | 108.6 | 108.6 | 94.4 |
| 1 | 129.5 | 166.0 | 134.6 | 131.4 | 97.6 |
| 1.5 | 161.9 | 181.3 | 163.3 | 153.7 | 98.1 |
| 2 | 189.6 | 189.0 | 182.4 | 170.2 | 96.5 |
| 2.5 | 216.1 | 191.7 | 205.7 | 188.7 | 93.3 |
| 3 | 235.0 | 199.0 | 228.0 | 209.4 | 89.1 |
| 3.5 | 260.6 | 207.7 | 254.1 | 225.4 | 90.1 |
| 4 | 273.8 | 205.4 | 278.5 | 239.7 | 91.7 |
| 4.5 | 289.1 | 206.4 | 305.0 | 254.6 | 88.0 |
| 5 | 305.1 | 203.7 | 326.3 | 257.8 | 90.7 |
| 5.5 | 319.5 | 212.0 | 345.4 | 264.1 | 94.4 |
| 6 | 342.7 | 199.0 | 371.9 | 275.8 | 93.9 |
| 6.5 | 352.7 | 206.0 | 378.8 | 282.2 | 96.5 |
| 7 | 360.7 | 200.0 | 389.5 | 289.1 | 94.4 |
| 7.5 | 361.7 | 203.0 | 399.0 | 292.8 | 95.4 |
| 8 | 371.2 | 199.0 | 408.6 | 289.1 | 99.2 |
| 8.5 | 383.5 | | 426.1 | 284.3 | 93.9 |
| 9 | 389.8 | | 418.7 | 290.7 | 93.9 |
| 9.5 | 389.1 | | 433.0 | 293.9 | 101.3 |
| 10 | 393.7 | | 436.2 | 293.9 | 100.2 |
| 10.5 | 395.2 | | 429.3 | 287.5 | 94.4 |
| 11 | 385.1 | | 438.9 | 290.7 | 97.0 |
| 11.5 | 395.2 | | 444.2 | 294.9 | 97.0 |
| 12 | 393.5 | | 440.4 | 290.7 | 100.2 |
| 12.5 | 393.7 | | 445.8 | 283.3 | 95.4 |

## Claims

1. The use of an additive composition consisting of an oil soluble or dispersible, metal salt additive and an oil soluble nitrogen-containing ashless detergent additive wherein the metal is an alkali metal, alkaline earth metal, Group IVB, VIIB, VIII, IB or IIB metal of rare earth metal, in a fuel having a boiling range and viscosity suitable for use in a diesel-type compression ignition engine, to improve the operation of a diesel exhaust particulate trap,
wherein the metal salt and ashless detergent additives are present in the fuel in such amounts as to provide 1 to 25 ppm metal and 10 to 500 ppm of detergent additive, and are co-operatively effective in improving the operation of the diesel exhaust particulate trap, as observed by a reduction in back-pressure build-up when the fuel containing the additive composition is used in a diesel engine equipped with said trap.

2. The use of claim 1, wherein the metal salt is a sulfonate, salicylate, naphthenate or neodecanoate.

3. The use of any one of the preceding claims, wherein the ashless detergent additive is a polyisobutenyl succinimide wherein the polyisobutenyl has an Mn of 400-2500.

4. The use of any one of the preceding claims, wherein there is present 10-200 ppm of the ashless detergent.

5. The use of any one of the preceding claims, wherein the metal salt is a carboxylate or complex derived from a compound of the formula: 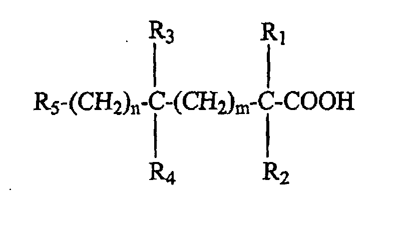 where R₁, R_{2,} R₃ and R₄ represent hydrogen or a hydrocarbyl having 1-30 carbon atoms (C₁-C₃₀), but at least two of R₁, R₂, R₃ or R₄ are C₁-C₃₀ hydrocarbyl; R₅ is a hydrocarbyl having 1 to 120 carbon atoms and m and n may each be zero or an integer such that the total number of carbon atoms in the carboxylate is not more than 125.

6. The use of any one of the preceding claims, wherein the metal is calcium, iron or cerium.

## Patentansprüche

1. Verwendung einer Additivzusammensetzung, die aus öllöslichem oder -dispergierbarem Metallsalzadditiv und öllöslichem stickstoffhaltigem Detergensadditiv besteht, wobei das Metall ein Alkalimetall, Erdalkalimetall, Gruppe IVB-, VIIB-, VIII-, IB- oder IIB-Metall oder Seltenerdmetall ist, in einem Brennstoff mit einem Siedebereich und einer Viskosität, die zur Verwendung in einem kompressionsgezündeten Motor vom Dieseltyp geeignet sind, zum Verbessern des Betriebs eines Dieselabgaspartikelfilters, wobei die Metallsalz- und aschefreien Detergensadditive in dem Brennstoff in solchen Mengen vorliegen, dass sie 1 bis 25 ppm Metall und 10 bis 500 ppm Detergensadditiv bereitstellen, und zusammenwirkend zum Verbessern des Betriebs des Dieselabgaspartikelfilters wirksam sind, wie durch eine Verminderung des Aufbaus von Gegendruck festgestellt wird, wenn der Brennstoff, der die Additivzusammensetzung enthält, in einem Dieselmotor, der mit der Falle ausgerüstet ist, verwendet wird.

2. Verwendung nach Anspruch 1, bei der das Metallsalz ein Sulfonat, Salicylat, Naphthenat oder Neodecanoat ist.

3. Verwendung nach einem der vorhergehenden Ansprüche, bei der das aschefreie Detergensadditiv Polyisobutenylsuccinimid ist, bei dem das Polyisobutenyl ein Molekulargewicht (Mn) von 400 - 2500 besitzt.

4. Verwendung nach einem der vorhergehenden Ansprüche, bei der 10 bis 200 ppm des aschefreien Detergens vorliegen.

5. Verwendung nach einem der vorhergehenden Ansprüche, bei der das Metallsalz ein Carboxylat oder Komplex ist, das bzw. der von einer Verbindung der Formel 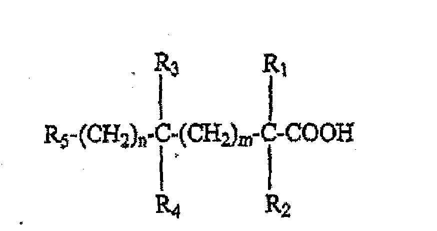 abgeleitet ist, in der R₁, R₂, R₃ und R₄ Wasserstoff oder einen Kohlenwasserstoffrest mit 1 bis 30 Kohlenstoffatomen (C₁ bis C₃₀) wiedergeben, jedoch mindestens zwei von R₁, R₂, R₃ oder R₄ C₁- bis C₃₀-Kohlenwasserstoff sind, R₅ ein Kohlenwasserstoffrest mit 1 bis 120 Kohlenstoffatomen ist und m und n jeweils 0 oder eine solche Zahl sein können, dass die Gesamtzahl an Kohlenstoffatomen in dem Carboxylat nicht mehr als 125 beträgt.

6. Verwendung nach einem der vorhergehenden Ansprüche, bei der das Metall Calcium, Eisen oder Cer ist.

## Revendications

1. Utilisation d'une composition d'additifs consistant en un additif du type sel métallique soluble ou dispersable dans l'huile et un additif détergent sans cendre azoté soluble dans l'huile, où le métal est un métal alcalin, un métal alcalinoterreux, un métal du Groupe IVB, VIIB, VIII, IB ou IIB ou un métal faisant partie des terres rares, dans un carburant ayant une plage d'ébullition et une viscosité convenables à des fins d'utilisation dans un moteur à allumage par compression de type diesel, pour améliorer le fonctionnement d'un piège à particules de système d'échappement de moteur diesel,
dans laquelle l'additif du type sel métallique et l'additif détergent sans cendre sont présents dans le carburant en des quantités choisies de manière à fournir 1 à 25 ppm de métal et 10 à 500 ppm d'additif détergent, et sont efficaces en coopération pour améliorer le fonctionnement du piège à particules de système d'échappement de moteur diesel, de la manière observée par une réduction de l'accumulation de contre-pression lorsque le carburant contenant la composition d'additifs est utilisé dans un moteur diesel équipé dudit piège.

2. Utilisation suivant la revendication 1, dans laquelle le sel métallique est un sulfonate, salicylate, naphténate ou néodécanoate.

3. Utilisation suivant l'une quelconque des revendications précédentes, dans laquelle l'additif détergent sans cendre est un polyisobutényl-succinimide dans lequel le groupe polyisobutényle a une valeur de Mn de 400 à 2500.

4. Utilisation suivant l'une quelconque des revendications précédentes, dans laquelle sont présentes 10 à 200 ppm du détergent sans cendre.

5. Utilisation suivant l'une quelconque des revendications précédentes, dans laquelle le sel métallique est un carboxylate ou complexe dérivé d'un composé de formule : 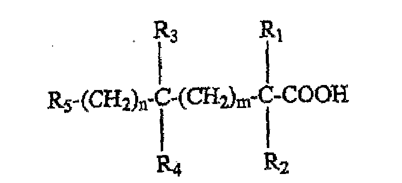 dans laquelle R₁, R₂, R₃ et R₄ représentent un atome d'hydrogène ou un groupe hydrocarbyle ayant 1 à 30 atomes de carbone (C₁ à C₃₀), mais au moins deux de R₁, R₂, R₃ ou R₄ représentent un groupe hydrocarbyle en C₁ à C₃₀ ; R₅ représente un groupe hydrocarbyle ayant 1 à 120 atomes de carbone et m et n peuvent être chacun égaux à zéro ou à un nombre entier tel que le nombre total d'atomes de carbone dans le carboxylate soit non supérieur à 125.

6. Utilisation suivant l'une quelconque des revendications précédentes, dans laquelle le métal est le calcium, le fer ou le cérium.
